# EUROPÄISCHE PATENTSCHRIFT

(11) **EP 2 139 542 B1**
(45) Veröffentlichungstag und Bekanntmachung des Hinweises auf die Patenterteilung: **29.05.2013**
(21) Anmeldenummer: 08734789.4
(22) Anmeldetag: 27.03.2008
(51) Int. Cl.: A61M 5/30, A61M 5/20

(54) **EINWEGINJEKTOR MIT MINDESTENS EINEM ZUGHAKEN UND EINEM SCHIEBEKEILGETRIEBE ZUM ENTSICHERNDEN LÖSEN EINES SPERRELEMENTS**
DISPOSABLE INJECTOR COMPRISING AT LEAST ONE DRAW HOOK AND A SLIDING WEDGE-TYPE GEAR FOR UNLOCKING A LOCKING ELEMENT
INJECTEUR À USAGE UNIQUE COMPRENANT AU MOINS UN CROCHET DE TRACTION ET UN MÉCANISME À CLAVETTE COULISSANTE SERVANT À DÉVERROUILLER UN ÉLÉMENT DE BLOCAGE

(30) Priorität: 19.04.2007 DE 102007018868
(43) Veröffentlichungstag der Anmeldung: 06.01.2010
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Siemund, Volker
(86) Internationale Anmeldenummer: PCT/EP2008/002391
(87) Internationale Veröffentlichungsnummer: WO 2008/128615

(56) Entgegenhaltungen:
- WO-A-2006/088513
- WO-A-2008/089886
- DE-A1-102004 060 146
- GB-A- 2 404 865
- US-A- 5 358 489

## Beschreibung

Die Erfindung betrifft einen Einweginjektor mit einem Gehäuse, in dem oder an dem - jeweils zumindest bereichsweise - mindestens ein mechanischer Federenergiespeicher, mindestens eine - zumindest zeitweise wirkstoffbefüllbare - Zylinder-Kolben-Einheit, mindestens ein Kolbenbetätigungsstempel und mindestens eine Auslöseeinheit angeordnet ist, wobei der Federenergiespeicher mindestens ein vorgespanntes Federelement umfasst, wobei zumindest ein Teil des Kolbenbetätigungsstempels zwischen dem Federenergiespeicher und dem Kolben der Zylinder-Kolben-Einheit positioniert ist und wobei der federbelastete Kolbenbetätigungsstempel mindestens einen zumindest bereichsweise querbeweglichen Zugstab aufweist, der mittels eines Abstützabschnitts den gespannten Federenergiespeicher an mindestens einer Auflagefläche des Gehäuses abstützt.

Aus der DE 36 44 984 A1, der US 5,358,489 und der DE 10 2004 060 146 A1 ist eine mit einer Injektionsnadel ausgestattete selbsttätige Injektionsvorrichtung bekannt, bei der die Injektionsnadel mittels eines mechanischen Federspeichers ausgefahren wird und zeitgleich der in der Vorrichtung gelagerte Injektionsstoff ausgetragen wird. Das im Federspeicher vorgespannte Federelement wird in der zuvor beschriebenen Weise im Gehäuse der Injektionsvorrichtung gehalten. Allerdings wird der Zugstab mittels eines separat entgegen der Auslöserichtung zu entfernenden knopfförmigen Sperrelements gesichert.

Der vorliegenden Erfindung liegt daher die Problemstellung zugrunde, einen modular aufgebauten Einweginjektor zu entwickeln, der bei geringer Baugröße nur wenige Bauteile aufweist und bei einfacher Handhabung eine sichere Lagerung und Funktion gewährleistet.

Diese Problemstellung wird mit den Merkmalen des Hauptanspruches gelöst. Dazu weist die Auslöseeinheit mindestens einen Auslöseschieber, mindestens einen am Gehäuse sich abstützenden Auslösehebel und ein den Zugstab in Sperrstellung haltendes Sperrelement auf. Der oder die Auslösehebel sind durch eine Schiebe - oder Schwenkbewegung des Auslöseschiebers so verlagerbar, dass das Sperrelement den Zughaken entsichert und den Abstützabschnitt von der Auflagefläche schiebt.

Mit der Erfindung wird ein beispielsweise nadelfreier Einmalinjektor vorgestellt, dessen Kolbenbetätigungsstempel oder Teile hiervon bei einem Auslösevorgang des Einweginjektors eine Bewegung erfährt, die quer zur Längsrichtung und/oder quer zur Mittellinie des Einweginjektors orientiert ist. Dazu liegen zum Vorspannen und Halten des Federenergiespeichers ein oder mehrere Teile des Kolbenbetätigungsstempels mit mindestens einem Umgriff oder einem Haken am Gehäuse oder einem am Gehäuse angeordneten Bauteil an. Zum Auslösen des Einweginjektors werden - nach einem automatischen Entsichern - die Umgriffe oder Haken von ihrer gehäuseseitigen Auflagefläche heruntergeschoben, so dass sich der Kolbenbetätigungsstempel unter der Wirkung des Federenergiespeichers zumindest annähernd parallel zur Mittellinie des Einweginjektors bewegen kann.

Die von einem Druckknopf abgeleitete Längsbewegung des auslösenden Bauteils kann auch mittels einer Drehbewegung gegenüber dem Injektorgehäuse erfolgen. Dazu wird das auslösende Bauteil über ein Schraubgewinde im Injektorgehäuse gelagert. Das Schraubgewinde und dieses Bauteil haben eine Mittellinie, die deckungsgleich zur Mittellinie des Injektorgehäuses ist. Durch eine entsprechende Drehbewegung wird das auslösende Bauteil in das Injektorgehäuse hineinbewegt. Dabei entsteht die für das Entsichern und Auslösen erforderliche Längsbewegung.

Die für das Entsichern und Auslösen notwendige Bewegung kann auch eine reine Schwenkbewegung sein. Dazu kann z.B. an der Innenwandung des auslösenden Bauteils eine Nocken- oder Exzenterstruktur angeformt sein, die durch das Schwenken oder Drehen des auslösenden Bauteils bestimmte Elemente, z.B. Auslösehebel der Auslöseeinheit bewegt.

Weitere Einzelheiten der Erfindung ergeben sich aus den Unteransprüchen und den nachfolgenden Beschreibungen von einigen schematisch dargestellten Ausführungsbeispielen.
- Figur 1:: Einweginjektor mit einem Zugstab;
- Figur 2:: wie Figur 1, jedoch entsichert und betätigt;
- Figur 3:: wie Figur 2, jedoch nach dem Medikamentenausstoß;
- Figur 4:: Einweginjektor mit außermittigem Zugstab;
- Figur 5:: wie Figur 4, jedoch entsichert und betätigt;
- Figur 6:: wie Figur 5, jedoch nach dem Medikamentenausstoß;
- Figur 7:: Einweginjektor mit separaten Auslösehebeln;
- Figur 8:: wie Figur 7, jedoch nach dem Lösen des Sperrelements;
- Figur 9:: wie Figur 8, jedoch betätigt;
- Figur 10:: wie Figur 9, jedoch nach dem Medikamentenausstoß;
- Figur 11:: separater Ring mit angeformten Auslösehebeln;
- Figur 12:: Einweginjektor mit am Gehäuse angeformten Auslösehebeln;
- Figur 13:: wie Figur 12, jedoch nach dem Lösen des Sperrelements;
- Figur 14:: wie Figur 13, jedoch betätigt;
- Figur 15:: wie Figur 14, jedoch nach dem Medikamentenausstoß;
- Figur 16:: dimetrische Ansicht des Gehäuses ohne Sicherungskappe, Stützhülse und Zylinder-Kolben-Einheit;
- Figur 17:: Schnitt des Gehäuses im Auslösebereich;
- Figur 18:: Ausschnitt zu Figur 12.

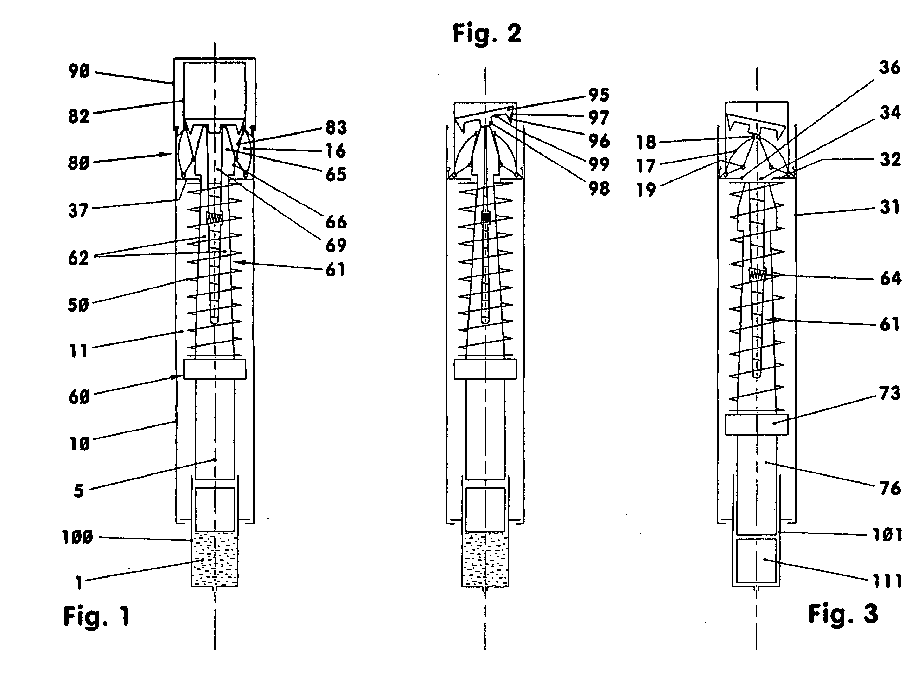

Die Figuren 1 bis 3 zeigen das vereinfachte Prinzip eines Einweginjektors mit einem dauergeladenen Federenergiespeicher. Der Einweginjektor besteht aus einem Gehäuse (10), einer z.B. befüllten Zylinder-Kolben-Einheit (100), einem Kolbenbetätigungsstempel (60) mit einem Zugstab (61) und einer Schraubendruckfeder (50) als Federspeicher. Der Zugstab (61) umfasst mehrfach geschlitzte Zughaken (62). Zudem sitzt auf dem Gehäuse (10) eine Auslöseeinheit (80), in der ein Auslöseelement (82) und eine Sicherungskappe (90) angeordnet sind.

Das Gehäuse ist ein topfförmiger, oben und unten offener Hohlkörper mit einem Zwischenboden (32). Der Zwischenboden (32) hat z.B. eine mittige Öffnung (34), durch die nach Figur 1 die Zughaken (62) hindurchgesteckt sind. Die einzelnen Zughaken (62) liegen mit ihren Abstützabschnitten (65) auf der Auflagefläche (37) des Gehäuses (10) auf.

Der Kolbenbetätigungsstempel (60) ist in drei Bereiche aufgeteilt. Der untere Bereich ist der Kolbenschieber (76). Sein Durchmesser ist etwas kleiner als der Innendurchmesser des Zylinders (101) der Zylinder-Kolben-Einheit (100). Die untere Stirnfläche des Kolbenschiebers (76) wirkt direkt auf den Kolben (111).

Der mittlere Bereich ist der Stempelteller (73). Der Stempelteller (73) ist eine flache, zumindest bereichsweise zylindrische Scheibe, deren Außendurchmesser einige Zehntel Millimeter kleiner ist als der Innendurchmesser des Gehäuses (10) im Mantelbereich (31).

Der obere Bereich ist der z.B. aus zwei einander gegenüberliegenden, elastischen Zughaken (62) bestehende Zugstab (61). Beide Zughaken (62) liegen Rücken an Rücken, wobei sie versuchen, sich blattfederartig auseinander zu drücken, so dass sie am Rand (36) der Öffnung (34) z.B. unter Vorspannung anliegen. Ihre Federrichtung wird symbolisiert durch eine ersatzweise zwischen ihnen querliegende Schraubendruckfeder (64).

Selbstverständlich kann eine derartige Schraubendruckfeder (64) auch real verwendet werden, sofern z.B. die Zughaken (62) am Stempelteller (73) mittels Schwenkgelenke angelenkt werden. Die Schwenkachsen dieser Schwenkgelenke lägen dann quer zur Gehäusemittellinie (5) und normal zur Darstellungsebene nach den Figuren 1 bis 3.

Im unteren Teil des Gehäuses (10) ist die Zylinder-Kolben-Einheit (100) befestigt. Die Zylinder-Kolben-Einheit (100) besteht hier aus einem mit einer Injektionslösung (1) befüllten Zylinder (101), in dem ein Kolben (111) in der hinteren Position sitzt. Oberhalb des Kolbens (111) ist im Gehäuse (10) der Kolbenbetätigungsstempel (60) z.B. so angeordnet, dass er den Kolben zwar nicht berührt, jedoch mit seinem unteren Ende im oberen Bereich des Zylinders (101) seitlich geführt wird.

Zwischen dem Stempelteller (73) und dem Zwischenboden (32) des Gehäuses (10) sitzt vorgespannt die Schraubendruckfeder (50).

Im oberen Bereich des Gehäuses (10) sitzt unter einer Sicherungskappe (90) die Auslöseeinheit (80) mit einem Auslöseschieber (82), mehreren Auslösehebeln (16) und einem Sperrelement (95).

Das Sperrelement (95) ist ein tellerförmiges Drehteil mit einem zentralen Sperrzapfen (98). Das Sperrelement (95) hat einen Rand (96), der sich in die gleiche Richtung erstreckt wie der Sperrzapfen (98). Der Rand (96) hat eine kegelstumpfmantelförmige Außenwandung (97), deren theoretische Spitze vor dem zentralen Sperrzapfen liegt. Der Spitzenwinkel der Außenwandung (97) liegt zwischen 25 und 100 Winkelgraden. Selbstverständlich kann das Sperrelement, z.B. bei zwei oder vier Zughaken (62), auch eine vier- oder mehreckige Platte sein, deren Außenwandung die Gestalt eines Pyramidenstumpfes hat. In diesem Fall hat der Sperrzapfen (98) auch einen rechteckigen Querschnitt.

Nach Figur 1, vgl. auch Figur 2, liegt das Sperrelement (95) auf den Zughaken (62) auf, wobei der Sperrzapfen (98) im oberen Bereich des zwischen den Zughaken (62) gelegenen Schlitzes (69) sichernd eingeklemmt ist. Der Sperrzapfen (98) hat hier z.B. einen rechteckigen Querschnitt. Er blockiert die Zughaken (62) mechanisch in ihrer Sperrstellung.

An den gegenüberliegenden Bereichen der Außenwandung (97) des Sperrelements (95) liegen die im Auslösebereich angeordneten Auslösehebel (16) mit ihren oberen Anlagezonen (18) an. Ggf. gleichzeitig berühren die unteren Anlagezonen (19) der Auslösehebel (16) die obere Außenkontur (66) der Zughaken (62). Des Weiteren stützt sich an den am Zwischenboden gelagerten Auslösehebeln (16) der topfförmige Auslöseschieber (82) ab. Letzterer ist im Bereich des oberen Randes des Gehäuses (10) vorerst formschlüssig verrastet. Hierbei liegt er mit einer Innenkante (83) an der jeweiligen Außenkontur (17) der einzelnen Auslösehebel (16) an.

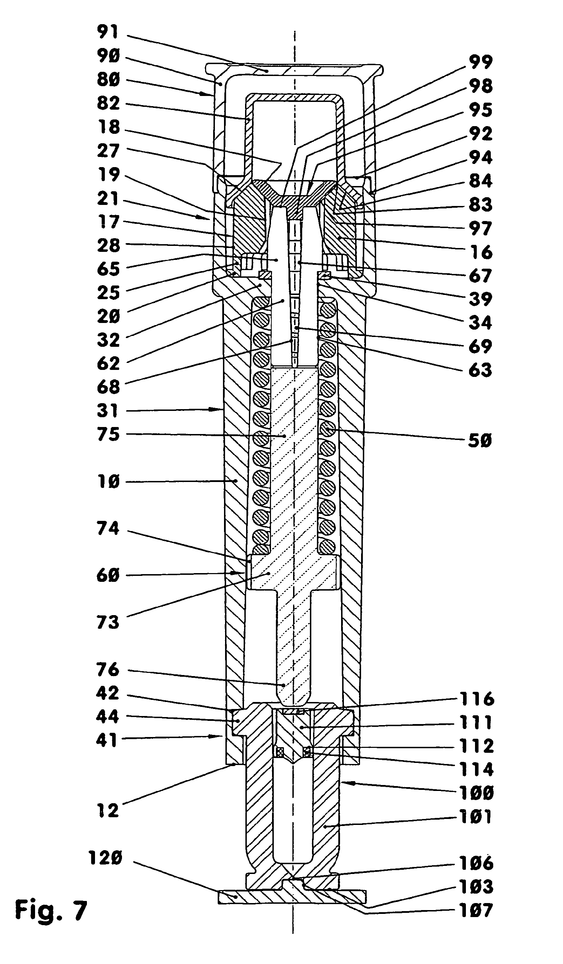

Ggf. können die Auslösehebel (16) an einem ringartigen Bauteil (20), vgl. Figur 7, befestigt sein. Dieses Bauteil (20) liegt dann beispielsweise auf dem Zwischenboden (32) auf. Von ihm ausgehend stehen die Auslösehebel (16) nach oben ab. Die zwischen je zwei Auslösehebeln gelegenen Abschnitte des ringartigen Bauteils (20) können u.a. als Torsionsfeder dienen.

Die Außenkontur (17) und die Anlagezonen (18, 19) sind in der Regel sphärisch gekrümmt. Es sind jedoch auch Flächen (17-19) denkbar, die zumindest bereichsweise eben bzw. plan gestaltet sind.

Der Auslöseschieber (82), der auf das obere Ende des Gehäuses (10) längsverschiebbar und vorläufig verrastet aufgesteckt ist, liegt an der z.B. zylindermantelförmigen Innenfläche des Auslösebereichs des Gehäuses (10) gleitfähig an.

Zum Entsichern des Einweginjektors wird die Sicherungskappe (90), vgl. Figur 1, vom Gehäuse (10) abgezogen. Anschließend wird der Einweginjektor auf die Injektionsstelle aufgesetzt. Zum Auslösen wird z.B. mit einem Finger der den Einweginjektor haltenden Hand der Auslöseschieber (82) - wie ein Druckknopf - linear in das Gehäuse (10) gedrückt. Die Innenkante (83) des Auslöseschiebers (82) drückt die Auslösehebel (16) über ihre Außenkontur mit der oberen Anlagezone (18) gegen die Außenwandung (97) des Sperrelements (95). Das Sperrelement (95) wird zwangsweise nach oben gepresst, wodurch der Sperrzapfen (98) - entgegen der Bewegungsrichtung des Auslöseschiebers (82) - aus dem Schlitz (69) springt. Zugleich, oder auch geringfügig später, legt sich die untere Anlagezone (19) an der Außenkontur (66) der Zughaken (62) an. Durch das zunehmende Schwenken der Auslösehebel (16) - unter dem Schub des Auslöseschiebers (82) - werden die Abstützabschnitte (65) der Zughaken (62) gegen die Wirkung des Federelements (64) zur Mittellinie (5) hin gebogen. In der Folge rutscht der Abstützabschnitt (65) - unter der Wirkung der Schraubendruckfeder (50) - durch die Öffnung (34) in das Innere (11) des Gehäuses (10). Hierbei wird die Zylinder-Kolben-Einheit (100) entleert, vgl. Figur 3.

Sobald die z.B. elastisch verformten Zughaken (62) als Teile des Kolbenbetätigungsstempels (60) das Innere (11) des Gehäuses (10) erreicht haben, federn sie z.B. wieder auseinander.

Die Art der Auslösung ist nicht auf die hier beschriebene Variante beschränkt. Anstelle des längs verschiebbaren Auslöseschiebers (82) kann z.B. u.a. ein Exzentergetriebe, ein Schraubgetriebe oder ein weiteres Hebelgetriebe treten.

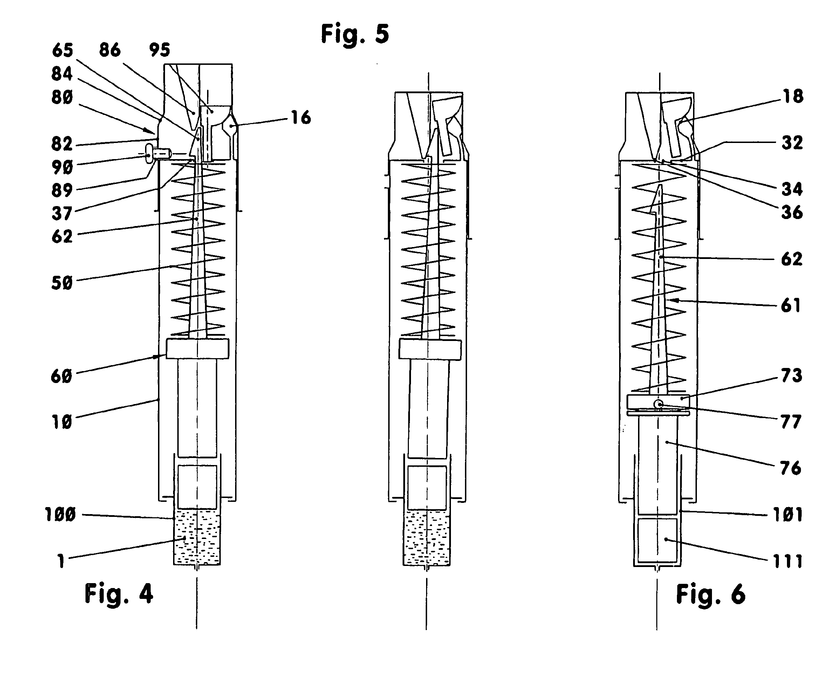

Die Figuren 4 bis 6 zeigen die Skizzen eines Einweginjektors, der einen außermittigen hakenförmig gestalteten Zugstab (62) aufweist. Letzterer durchdringt eine außermittige Öffnung (34) des Bodens (32). Der Zugstab (62) liegt mit seinem Abstützabschnitt (65) auf der Auflagefläche (37) auf. Vom Boden (32) steht nach oben ein Auslösehebel (16) ab.

Auf dem Gehäuse (10) sitzt längsverschiebbar das kappenförmige Auslöseelement (82). Das Auslöseelement (82) hat einen kegeligen, einen kegelstumpfförmigen oder einen pyramidalen Bereich (84) und trägt in seinem Innenraum zusätzlich einen Auslösekeil (86). Seine Wandung weist eine Ausnehmung (89) auf, in der nach Figur 4 optional ein z.B. pilzförmiges Sicherungselement (90) angeordnet ist. Das Sicherungselement (90) liegt auf der Auflagefläche (37) auf.

Zwischen dem Zugstab (62) und dem Auslösehebel (16) befindet sich in der Auslöseeinheit (80) ein Sperrelement (95). Es stützt sich linksseitig am Auslösekeil (86) und rechtsseitig auf der oberen Anlagezone (18) des Auslösehebels (16) ab. Zugleich steckt ein Teil des Sperrelements (95) - den Zugstab (62) blockierend - in der Ausnehmung (34).

Zum Entsichern des Einweginjektors wird zunächst das Sicherungselement (90) aus dem Auslöseelement (82) herausgezogen. Anschließend wird zum Auslösen des Einweginjektors das Auslöseelement (82) durch ein druckknopfartiges Drücken betätigt. Das Auslöseelement (82) gleitet dabei an der Außenwandung des Gehäuses (10) entlang. Hierbei kontaktiert zuerst der Bereich (84) den Auslösehebel (16) und drückt diesen über die Anlagezone (16) gegen das Sperrelement (95). Letzteres weicht nach oben aus und gibt hierbei die Ausnehmung (34) frei. Anschließend legt sich der Auslösekeil (86) am Abstützabschnitt (65) des Zugstabs (62) an und schiebt diesen in die Ausnehmung (34), vgl. Figur 5. Der so freigegebene Kolbenbetätigungsstempel (60) schnellt abwärts und entleert hierbei die Zylinder-Kolben-Einheit (100).

Bei diesem und bei den anderen hier vorgestellten Prinzipien kann der Kolbenbetätigungsstempel (60) einen separaten Kolbenschieber (76) haben. Hierzu ist er dann an der Innenwandung des Gehäuses (10) geführt und/oder durch ein Gelenk (77) am Stempelteller (73) gelagert, vgl. Figur 6.

Es ist auch möglich, den Kolbenschieber (76) als Kolbenstange am Kolben (111) anzuformen und dabei die Kolbenstange nur durch den Kolben (111) und/oder durch eine - z.B. bereichsweise - Anlage an der Innenwandung des Zylinders (101) zu führen. Selbstverständlich können sich der Kolbenschieber (76) und die Kolbenstange den Raum zwischen dem Stempelteller (73) und dem Kolben (111) beliebig aufteilen.

Die Figuren 7 bis 11 zeigen eine Ausführungsform des in den Figuren 1 bis 3 beschriebenen Prinzips. Hier ist das tragende Bauteil das Gehäuse (10). Es hat eine weitgehend rohrförmige Gestalt und ist in drei Funktionsbereiche (21, 31, 41) aufgeteilt. Nach der Figur 7 ist der obere Bereich der Auslösebereich (21). An ihn schließt sich der Mantelbereich (31) an. Zwischen beiden Bereichen ist ein Zwischenboden (32) angeordnet, der zudem geringfügig radial über den Mantelbereich (31) übersteht. Der Zwischenboden (32) hat eine zentrale Ausnehmung (34), deren Durchmesser sich z.B. nach unten hin geringfügig weitet.

Im Auslösebereich (21) des Gehäuses (10) befindet sich auf dem Zwischenboden (32) ggf. eine formsteife z.B. metallische Lochscheibe, die hier beispielsweise versenkt um die Bohrung (34) angeordnet ist. Sie ist dort eingeklebt oder eingespritzt. Die Lochscheibe (39) schützt den Zwischenboden (32) vor Eindrückungen und/oder anderen Verformungen. Sie verhindert auch ein Verkleben der sich dort ansonsten kontaktierenden Bauteile (32) und (65).

Unterhalb des Mantelabschnitts (31) befindet sich der Fixierbereich (41) zur Aufnahme der einbaubaren Zylinder-Kolben-Einheit (100). Der ungeschlitzte Fixierbereich (41) ist Teil eines Bajonettverschlusses. Dazu sind an seiner Innenwandung zwei oder mehrere winkelförmige Kanäle (42) angeordnet. Die Kanäle (42) führen von der unteren Gehäusestirnseite (12) aus vertikal nach oben und gehen nach wenigen Millimetern Länge jeweils in einen kurzen horizontalen Kanalabschnitt über.

Im Fixierbereich (41) ist der Zylinder (101) über z.B. zwei oder mehrere Bajonettzapfen (44) eingesetzt und fixiert. Ggf. befindet sich im horizontalen Kanalabschnitt oder an zumindest einem Teil der Bajonettzapfen (44) ein oder mehrere Rastelemente, die ein Lösen des Bajonettverschlusses - also ein Entfernen des Zylinders (101) - verhindern.

Der Zylinder (101) ist ein z.B. dickwandiger Topf. In der beispielsweise zylindrischen Bohrung des Zylinders (101) sitzt der stangenlose Kolben (111). Der Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. In der rückseitigen Stirnfläche des Kolbens (111) ist eine z.B. zylindrische Metallplatte (116) eingelassen.

Im Zentrum der Bohrung des Zylinders (101), dessen Zylinderboden der Kontur der vorderen Kolbenstirnseite zumindest annähernd angepasst ist, befindet sich eine kurze zylindrische, düsenartige Bohrung (106). Ihr Durchmesser beträgt ca. 0,1 bis 0,5 Millimeter. Diese Bohrung (106) ist ein- bis fünfmal so lang wie ihr Durchmesser. Sie endet in einer zylindrischen Ausnehmung (107) der bodenseitigen, äußeren Stirnfläche (103) des Zylinders (101).

Zwischen dem Kolben (111) und dem Auslösebereich (21) ist der Federenergiespeicher (50) bzw. die Antriebseinheit des Einweginjektors angeordnet. Der Federenergiespeicher (50) ist eine Schraubendruckfeder, die auf einem Kolbenbetätigungsstempel (60) mit vier Zughaken (62) angeordnet ist. Mittels der Abstützabschnitte (65) der Zughaken (62) sitzt die Schraubendruckfeder (50) gespannt im Gehäuse (10). Letztere stützt sich zwischen der Innenseite des Zwischenbodens (32) und einer oberen Stirnseite des Kolbenbetätigungsstempels (60) ab.

Der Kolbenbetätigungsstempel (60) ist dabei in drei Bereiche aufgeteilt. Der untere Bereich ist der Kolbenschieber (76), der mittlere Bereich ist der das Federelement (50) abstützende Stempelteller (73) und der obere Bereich ist ein z.B. zylindrischer Abschnitt (75), an den sich das Bündel aus z.B. vier Zughaken (62) anschließt. Der Abschnitt (75) und das Bündel aus Zughaken (62) haben im Mantelbereich (31) des Gehäuses (10) zumindest annähernd eine zylindrische Hüllfläche (63), d.h. ihre äußeren Wandungen haben die Krümmung eines Zylindermantelbereiches. Der Durchmesser der Hüllfläche (63) ist kleiner als der kleinste Innendurchmesser des Federelements (50).

Der Abstützabschnitt (65) hat als Hüllfläche hier bereichsweise einen Kegelstumpfmantel und bereichsweise einen Zylinder. Beide Bereich sind z.B. gleich lang.

Die inneren Wandungen der Zughaken (62) sind Teile einer kegelstumpfmantelförmigen Hüllfläche (68). Diese Hüllfläche (68) umgibt den zwischen den Zughaken gelegenen kegelstumpfmantelförmigen Hohlraum (67). Hierbei vergrößern sich die Querschnitte des Hohlraums (67) je weiter sie sich vom Abschnitt (75) entfernen. Die radialen, zwischen benachbarten Zughaken (62) liegenden Schlitze (69) vergrößern sich gemäß Figur 7 nach oben hin auf die ca. dreifache Breite.

Der Stempelteller (73) nach Figur 7 hat z.B. zwei einander gegenüberliegende Nuten (74).

Die Zughaken (62) stecken mit ihren Abstützabschnitten (65) im Auslösebereich (21). Die Abstützabschnitte (65) liegen gesichert auf dem Zwischenboden (32) auf.

Im Auslösebereich (21) sitzt als Teil einer Auslöseeinheit (80) ein Auslöseschieber (82) in seiner oberen Position unter einer Sicherungskappe (90). Der Auslöseschieber (82) ist ein topfförmiger Körper, dessen vorderer Bereich sich trichterförmig aufweitet. Der Kegelspitzenwinkel dieses Bereiches beträgt hier z.B. 90 Winkelgrade. Die Innenwandung des trichterförmigen Bereiches dient als Gleitfläche (84), die an einer sog. Innenkante (83) endet. Dort geht die Gleitfläche (84) in einen zumindest annähernd zylindrischen Bereich über. Der Hohlraum des zylindrischen Bereiches ist so groß gewählt, dass das Sperrelement (95) in jeder beliebigen Lage und bei jeder Auslösestellung des Auslöseschiebers (82) hineinpasst.

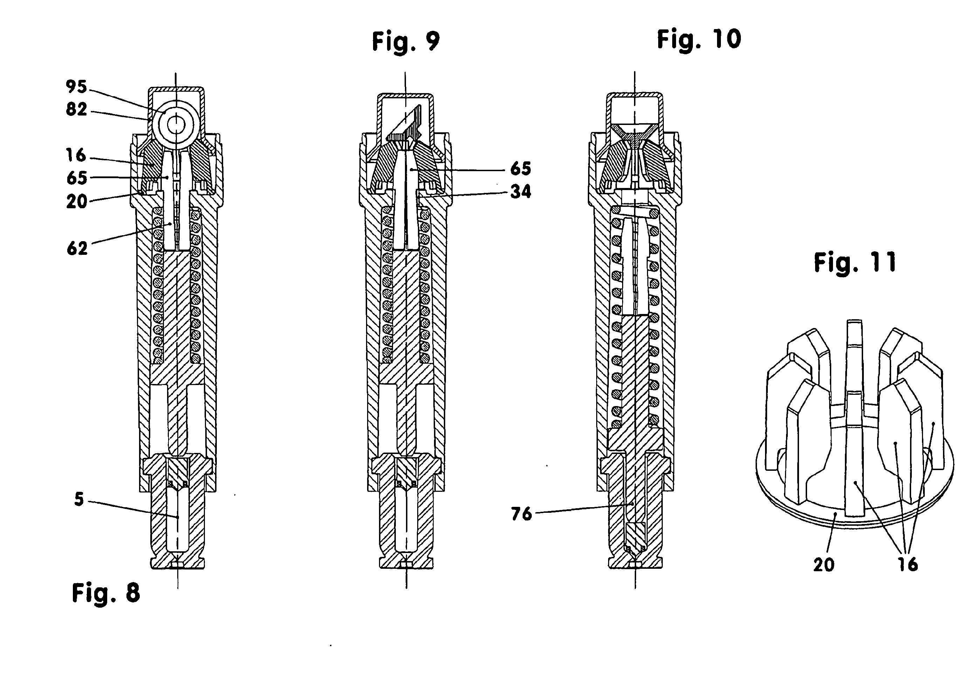

Der Auslöseschieber (82) liegt mit seiner Gleitfläche (84) auf einer Gruppe von acht Auslösehebeln (16) auf. Die Auslösehebel (16) sind an einem Ring (20) angeformt, vgl. Figur 11. Der Ring (20) liegt nach Figur 7 auf dem Zwischenboden (32) lose auf. Ggf. kann er auch am Gehäuse (10) festgeklemmt und/oder verrastet sein. Zumindest stützt sich der Ring (20) an der zumindest annähernd zylindrischen Innenwandung des Auslösebereichs (21) ab.

Die einzelne Auslösehebel (16) sind hier schmale z.B. sechseckige Platten, die über einen Fuß (25) am Ring (20) angeordnet sind. Die Auslösehebel (16) haben eine zweiteilige Außenkontur (17). Der obere Teil (27) liegt nach Figur 7 flächig an der Gleitfläche (84) an. Der untere Teil (28) verläuft z.B. geradlinig annähernd parallel zur Innenwandung des Auslösebereichs (21). Dabei endet dieser Teil (28) der Außenkontur (17) am Ring (20).

Zur Mittellinie (5) gewandt hat jeder Auslösehebel (16) zwei aktive Zonen. Diese sind eine obere (18) und eine untere Anlagezone (19). Die obere Anlagezone (18) ist - nach Figur 7 - gegenüber der Mittellinie (5) um 45 Winkelgrade geneigt. Sie kontaktiert das Sperrelement (95) an dessen kegelstumpfförmigen Außenwandung (97). Die untere Anlagezone (19) schließt mit der Mittellinie (5) einen Winkel von ca. einem Winkelgrad ein. Diese Zone hat nach Figur 7 keinen Kontakt zu den Zughaken (62).

Die Außenkonturteile (27, 28) und die Anlagezonen (18, 19) sind hier beispielsweise jeweils Teilflächen von Kegel- oder Zylindermänteln. Die Mittellinien dieser Mäntel sind mit der Mittellinie (5) deckungsgleich.

Die Teilung der auf dem Ring (20) sitzenden Auslösehebel (16) ist so gewählt, dass keiner der Auslösehebel (16) beim Auslösevorgang in einen der Schlitze (69) geraten kann. Ggf. wird dies auch durch eine besondere Formgebung des Schlitzes (69) oder der Auslösehebel (16) gewährleistet.

Das nach Figur 7 auf den Auslösehebeln aufliegende Sperrelement (95) hat eine trichterförmige Gestalt. Das Sperrelement (95) ist wie der Auslöseschieber (82) ein rotationssymmetrisches Bauteil. Es besteht aus einem Teller mit einem zentralen Sperrzapfen (98). Der Sperrzapfen (98) steckt im oberen Bereich des zwischen den Zughaken (62) gelegenen Hohlraumes (67). Der Teller hat an seiner Unterseite eine Stützfläche (99), mit der er auf den oberen Enden der Zughaken (62) aufliegt.

Die Sicherungskappe (90), die das obere Ende des Gehäuses (10) verschließt und den Auslöseschieber (82) vor einem ungewollten Betätigen schützt, hat ebenfalls eine topfförmige Gestalt. Sie hat einen fast ebenen Boden (91), der ringsherum über die Seitenwandung der Sicherungskappe (90) geringfügig übersteht, so dass die Kappe (90) auch mit dem Daumen der den Einweginjektor haltenden Hand - im Rahmen einer Einhandbedienung - weggesprengt werden kann. Zum Gehäuse (10) hin weist sie einen Absatz (92) auf, der in einem umlaufenden Raststollen (94) endet. Mittels des Absatzes (92) sitzt die Sicherungskappe (90) auf der oberen Stirnseite des Gehäuses (10) sicher auf. Der Raststollen (94) greift elastisch in eine Nut des Gehäuses (10) ein, um die Kappe (90) sicher und ggf. dicht am Gehäuse (10) zu fixieren.

Die Figur 8 zeigt den Einweginjektor mit entfernter Sicherungskappe (90) und einem teilbetätigten, also teilweise niedergedrückten Auslöseschieber (82). Durch die Teilbetätigung wird die obere Anlagezone (18) der Auslösehebel (16) gegen die Gleitfläche (84) des Sperrelements (95) gepresst, vgl. Figur 7. Die Anlagezone (18) gleitet entlang der Gleitfläche (84) zur Mittellinie (5) hin. Der einzelne Auslösehebel (16) schwenkt dabei jeweils um eine horizontale Achse, die im Bereich des jeweiligen Fußes (25) oder im Bereich des Ringes (20) liegt.

Als Folge der Bewegung der Auslösehebel (16) wird das Sperrelement (95) nach oben aus dem Hohlraum (67) geschoben. Die Auslösehebel (16) liegen nun an den Abstützabschnitten (65) der Zughaken (62) an. Durch ein weiteres Niederdrücken des Auslöseschiebers (82) wird die untere Anlagezone (19) gegen den Abstützabschnitt (65) gepresst. Dadurch werden die Zughaken (62) radial in Richtung Mittellinie (5) elastisch und/oder plastisch gebogen. Die Abstützabschnitte (65) werden auf dem Zwischenboden gleitend in Richtung auf die Mittellinie (5) verschoben. Der Spaltraum zwischen den einzelnen Zughaken (62) wird dabei zumindest im Bereich der Abstützabschnitte (65) weitgehend verbraucht, vgl. Figur 9. Sobald der maximale Außendurchmesser der Abstützabschnitte (65) kleiner ist als der Durchmesser der Bohrung (34), können sich die Zughaken (62) unter der Wirkung des Federelements (50) nach unten bewegen und den Kolben (111) mittels des Kolbenschiebers (76) verschieben, vgl. Figur 10.

In den Figuren 10 bis 18 wird eine Einweginjektor-Variante dargestellt, deren Auslösehebel (16) am Gehäuse (10) angeformt sind und die zur Einweginjektor-Mittellinie (5) hin elastisch federn. Das Gehäuse (10) ist im Wesentlichen ein glattes Rohr mit einem oben liegenden, ebenen Zwischenboden (32). Im Zwischenboden (32) ist zum Durchführen des Kolbenbetätigungsstempels (60) eine zentrale Bohrung (34) eingearbeitet.

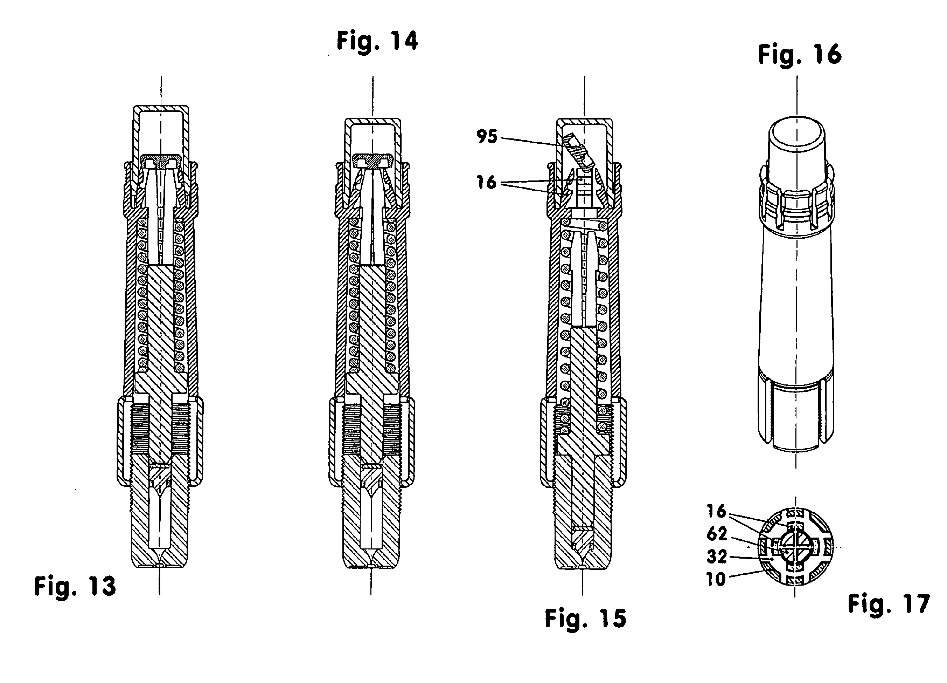

Im unteren Bereich des Gehäuses (10) befindet sich der Fixierbereich (41) zur Aufnahme der einbaubaren Zylinder-Kolben-Einheit (100). Der Fixierbereich (41) ist z.B. vierfach längsgeschlitzt, vgl. auch Figur 16. Die Innenwandung dieses Bereiches trägt z.B. ein Gewinde (46). Nach den Figuren 10 bis 15 ist der Fixierbereich (41) von einer am Gehäuse (10) verrasteten Stützhülse (49) umgeben.

In das Gewinde (46) ist eine Zylinder-Kolben-Einheit (100) eingeschraubt. Letztere besteht aus einem Zylinder (101) und einem Kolben (111). Der Zylinder (101) ist ein z.B. dickwandiger Topf, dessen ggf. zylindrische Außenwandung zumindest bereichsweise ebenfalls ein Gewinde (104) trägt.

Im Gehäuse (10) sitzt zwischen dem Zwischenboden (32) und der Zylinder-Kolben-Einheit (100) der Kolbenbetätigungsstempel (60). Sämtliche Bereiche (61, 73, 75, 76) des Kolbenbetätigungsstempels (60) sind zumindest annähernd aus Figur 7 bekannt.

Auch hier ist das hintere Ende des Gehäuses (10) mittels einer Sicherungskappe (90) verschlossen. Sie ist mit der aus Figur 7 weitgehend vergleichbar. Allerdings ist das Gehäuse im Auslösebereich (21) mehrfach geschlitzt, vgl. auch die Figuren 16 und 17, so dass die Sicherungskappe (90) außen bis zum Zwischenboden heruntergezogen ausgebildet ist.

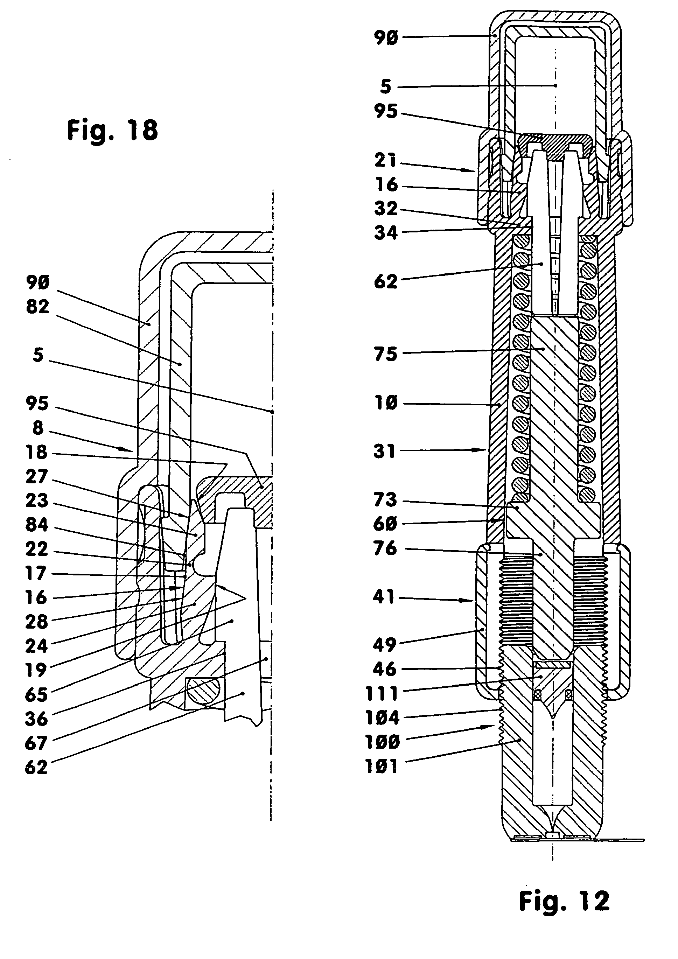

Bei dieser Variante werden beispielsweise vier breite, am Zwischenboden (32) angeformte Auslösehebel (16) verwendet. Die Auslösehebel (16), vgl. Figur 17, haben sichelförmige Querschnitte und verfügen jeweils über ein mittiges Gelenk (22), vgl. Figur 18. Letzteres trennt jeweils die obere Anlagezone (18) von der unteren Anlagezone (19) und den oberen Teil (27) der Außenkontur (17) von dem unteren Teil (28) dieser Kontur.

Der Auslöseschieber (82) hat an seinem unteren Rand als Fase eine breite, kegelstumpfmantelförmige Gleitfläche (84), deren Spitzenwinkel ca. 12 bis 16 Winkelgrade einnimmt. Diese Gleitfläche (84) liegt schon in der Sperrstellung (8) flächig am oberen Teil (27) der Außenkontur (17) an, vgl. Figur 18. Wird nun beim Auslösen der Auslöseschieber (82) durch Drücken betätigt, schwenken die oberen Bereiche (23) der Auslösehebel (16) unabhängig von den unteren Bereichen (24) der Auslösehebel (16) auf die Mittellinie (5) zu, wobei sie über ihre obere Anlagezone (18) das Sperrelement (95) nach oben drängen. Letzteres springt aus dem Hohlraum (67), vgl. Figur 13. Die oberen Bereiche (23) der Auslösehebel (16) verschwenken gegenüber den unteren Bereichen (24) an den elastischen Gelenken (22).

Bei einem kontinuierlichen Weiterbewegen des Auslöseschiebers (82) rutscht die Gleitfläche (84) über die Zone mit den Gelenken (22) hinweg und drängt nun die unteren Bereiche (24) der Auslösehebel (16) gegen die Abstützabschnitte (65) der Zughaken (62), vgl. Figur 14. Hierbei knicken die unteren Bereiche (24) am Zwischenboden (32) elastisch oder plastisch ab, jedoch ohne am Zwischenboden (32) abzureißen. Die untere Anlagezone (19) schieben die Abstützbereiche (65) über den Rand (36) in die Bohrung (34). Der Kolbenbetätigungsstempel (60) schnellt nach unten, vgl. Figur 15.

Bei dieser Variante können die Gleitfläche (84), die Anlagezonen (18, 19), die Außenkonturteile (27, 28) und die Außenwandung (97) auch sphärische Flächen sein.

Mit Ausnahme der Federelemente (50, 64), der Lochscheibe (39) und der ggf. verwendeten Metallplatte (116) des Kolbens (111) sind alle Teile des Einweginjektors aus Kunststoffen oder kunststoff- bzw. gummiähnlichen Werkstoffen gefertigt. Einzelne z.B. durch Hertzsche Flächenpressung hochbelastete Kunststoffbereiche können durch eine Keramisierung gepanzert sein.

### Bezugszeichenliste:

| | |
|---|---|
| 1 | Injektionslösung; Medikament |
| 5 | Mittellinie des Einweginjektors |
| 8 | Sperrstellung |
| | |
| 10 | Gehäuse, einteilig |
| 11 | Gehäuseinnenraum |
| 12 | Gehäusestirnseite, unten |
| | |
| 16 | Auslösehebel |
| 17 | Außenkontur |
| 18 | Innenkontur, oben; Anlagezone, oben |
| 19 | Innenkontur, unten; Anlagezone, unten |
| | |
| 20 | Ring |
| 21 | Auslösebereich |
| 22 | Gelenk, Filmgelenk |
| 23 | Bereich, oben |
| 24 | Bereich, unten |
| 25 | Fuß |
| 27 | Außenkontur, oberer Teil |
| 28 | Außenkontur, unterer Teil |
| | |
| 31 | Mantelbereich |
| 32 | Zwischenboden |
| 34 | Öffnung, Bohrung, Ausnehmung |
| 36 | Gehäusekante, Rand |
| 37 | Auflagefläche |
| 39 | Lochscheibe |
| | |
| 41 | Fixierbereich für die Zylinder-Kolben-Einheit |
| 42 | Kanäle, winkelförmig |
| 44 | Bajonettzapfen |
| | |
| 46 | Gewinde, Innengewinde |
| 49 | Stützhülse |
| | |
| 50 | Federelement, Schraubendruckfeder, Federenergiespeicher |
| | |
| 60 | Kolbenbetätigungsstempel |
| 61 | Zugstab |
| 62 | Zughaken |
| 63 | Hüllfläche, unten |
| 64 | Federelement, Schraubendruckfeder |
| 65 | Abstützabschnitt |
| 66 | Außenkontur, Hüllfläche, oben |
| 67 | Hohlraum zwischen den Zughaken |
| 68 | Hüllfläche, innen |
| 69 | Schlitze zwischen den Zughaken |
| | |
| 73 | Stempelteller |
| 74 | Nuten |
| 75 | Abschnitt, z.B. zylindrisch |
| 76 | Kolbenschieber |
| 77 | Gelenk |
| | |
| 80 | Auslöseeinheit |
| 82 | Auslöseschieber, Druckknopf auslösendes Bauteil |
| 83 | Innenkante |
| 84 | Gleitfläche, Bereich |
| 86 | Auslösekeil |
| 89 | Ausnehmung |
| 90 | Sicherungskappe |
| 91 | Boden |
| 92 | Absatz |
| 94 | Raststollen, Rastnasen |
| | |
| 95 | Sperrelement, tellerförmig |
| 96 | Rand |
| 97 | Außenwandung, kegelstumpfförmig |
| 98 | Sperrzapfen |
| 99 | Stützfläche |
| | |
| 100 | Zylinder-Kolben-Einheit |
| 101 | Zylinder |
| 103 | Stirnfläche |
| 104 | Gewinde |
| 106 | Bohrung, Düse |
| 107 | Ausnehmung in der Stirnfläche |
| | |
| 111 | Kolben |
| 112 | Ringnut |
| 114 | Dichtring |
| 116 | Metallplatte, magnetisch oder magnetisierbar |
| | |
| 120 | Schutzfolie, Klebeversiegelung |

## Patentansprüche

1. Einweginjektor mit einem Gehäuse (10), in dem oder an dem - jeweils zumindest bereichsweise - mindestens ein mechanischer Federenergiespeicher, mindestens eine - zumindest zeitweise wirkstoffbefüllbare - Zylinder-Kolben-Einheit (100), mindestens ein Kolbenbetätigungsstempel (60) und mindestens eine Auslöseeinheit (80) angeordnet ist, wobei der Federenergiespeicher (50) mindestens ein vorgespanntes Federelement umfasst, wobei zumindest ein Teil des Kolbenbetätigungsstempels (60) zwischen dem Federenergiespeicher (50) und dem Kolben (111) der Zylinder-Kolben-Einheit (100) positioniert ist und wobei der federbelastete Kolbenbetätigungsstempel (60) mindestens einen zumindest bereichsweise querbeweglichen Zugstab (61) aufweist, der mittels eines Abstützabschnitts (65) den gespannten Federenergiespeicher (50) an mindestens einer Auflagefläche (37) des Gehäuses (10) abstützt,
**dadurch gekennzeichnet,**
- **dass** die Auslöseeinheit (80) mindestens einen Auslöseschieber (82), mindestens einen am Gehäuse (10) sich abstützenden Auslösehebel (16) und ein den Zugstab (61) in Sperrstellung haltendes Sperrelement (95) aufweist,
- **dass** der oder die Auslösehebel (16) durch eine Längsschiebe - oder Schwenkbewegung des Auslöseschiebers (82) so verlagerbar sind, dass das Sperrelement (95) den Zugstab (61) entsichert und den Abstützabschnitt (65) von der Auflagefläche (37) schiebt.

2. Einweginjektor gemäß Anspruch 1, **dadurch gekennzeichnet, dass** der Zugstab (61) zusammen mit dem Abstützabschnitt (65) einen Zughaken (62) bildet, der in einer Sperrstellung die Gehäusekante (36) abstützend übergreift.

3. Einweginjektor gemäß Anspruch 2, **dadurch gekennzeichnet, dass** am Kolbenbetätigungsstempel (60) mindestens ein Bündel aus zwei oder mehr Zughaken (62) angeordnet ist.

4. Einweginjektor gemäß Anspruch 2, **dadurch gekennzeichnet, dass** am Gehäuse (10) oder an der Auslöseeinheit (80) ein Sperrelement (95) angeordnet ist, das den oder die Zughaken (62) in einer Sperrstellung sichert.

5. Einweginjektor gemäß Anspruch 1, **dadurch gekennzeichnet, dass** bei der Verwendung mehrerer Auslösehebel (16) diese an oder auf einem Ring (20) angeordnet sind.

6. Einweginjektor nach mindestes einem der vorangegangenen Ansprüche, **dadurch gekennzeichnet, dass** der Auslöseschieber (82) in Kombination mit dem oder den Auslösehebeln (16) ein Schiebekeilgetriebe bildet, wobei bei einer Längsbewegung des Auslöseschiebers (82) das Sperrelement (95) zwangsläufig eine Bewegung entgegen der vorgenannten Längsbewegung erfährt, während die Zughaken (62) quer zur Mittellinie (5) des Einweginjektors bewegt werden.

7. Einweginjektor gemäß Anspruch 2, **dadurch gekennzeichnet, dass** bei einem Kolbenbetätigungsstempel (60) mit mindestens zwei Zughaken (62), die Schwerpunkte der Abstützabschnitte (65) der Zughaken (62) aufgrund ihrer Fertigung im unverformten Zustand weiter auseinander liegen als bei einem im Einweginjektor eingebauten, nicht ausgelösten Kolbenbetätigungsstempel (60).

## Claims

1. Disposable injector with a housing (10) in which or on which are arranged, in each case at least in some areas, at least one mechanical spring energy reservoir, at least one cylinder/piston unit (100) that can be filled at least temporarily with active substance, at least one piston-actuating ram (60) and at least one trigger unit (80), in which the spring energy reservoir (50) comprises at least one pretensioned spring element, in which at least part of the piston-actuating ram (60) is positioned between the spring energy reservoir (50) and the piston (111) of the cylinder/piston unit (100), and in which the spring-loaded piston-actuating ram (60) has at least one tension bar (61) which is transversely movable at least in some areas and which, by means of a support portion (65), supports the tensioned spring energy reservoir (50) on at least one bearing surface (37) of the housing (10),
**characterized in that**
- the trigger unit (80) has at least one trigger slide (82), at least one trigger lever (16) bearing on the housing (10), and a catch element (95) that holds the tension bar (61) in the locked position, and
- the trigger lever or trigger levers (16) can be displaced by a longitudinal sliding movement or pivoting movement of the trigger slide (82) in such a way that the catch element (95) releases the tension bar (61) and slides the support portion (65) from the bearing surface (37).

2. Disposable injector according to Claim 1, **characterized in that** the tension bar (61) forms, together with the support portion (65), a tension hook (62) which, in a locked position, engages over the housing edge (36).

3. Disposable injector according to Claim 2, **characterized in that** at least one bundle of two or more tension hooks (62) is arranged on the piston-actuating ram (60).

4. Disposable injector according to Claim 2, **characterized in that** a catch element (95), that secures the tension hook or tension hooks (62) in a locked position, is arranged on the housing (10) or on the trigger unit (80).

5. Disposable injector according to Claim 1, **characterized in that**, when several trigger levers (16) are used, these are arranged on a ring (20).

6. Disposable injector according to at least one of the preceding claims, **characterized in that** the trigger slide (82) forms a spline gear in combination with the trigger lever or trigger levers (16), and, upon a longitudinal movement of the trigger slide (82), the catch element (95) necessarily executes a movement counter to the aforementioned longitudinal movement, while the tension hooks (62) are moved transversely to the centre line (5) of the disposable injector.

7. Disposable injector according to Claim 2, **characterized in that**, in a piston-actuating ram (60) with at least two tension hooks (62), the centres of gravity of the support portions (65) of the tension hooks (62) lie further apart from one another in the non-deformed state than they do in the case of a piston-actuating ram (60) fitted in the disposable injector and not triggered.p

## Revendications

1. Injecteur à usage unique comprenant un boîtier (10), dans lequel ou contre lequel - à chaque fois au moins par zones - sont disposés au moins un accumulateur d'énergie à ressort mécanique, au moins une unité cylindre-piston (100) - pouvant être au moins temporairement remplie de substance active -, au moins un poinçon d'actionnement de piston (60) et au moins une unité de déclenchement (80), l'accumulateur d'énergie à ressort (50) comprenant au moins un élément de ressort précontraint, au moins une partie du poinçon d'actionnement de piston (60) étant positionnée entre l'accumulateur d'énergie à ressort (50) et le piston (111) de l'unité cylindre-piston (100) et le poinçon d'actionnement de piston (60) sollicité par ressort présentant au moins une barre de traction (61) déplaçable transversalement au moins par zones, qui supporte, au moyen d'une portion d'appui (65), l'accumulateur d'énergie à ressort tendu (50) contre au moins une surface d'appui (37) du boîtier (10),
**caractérisé en ce que**
- l'unité de déclenchement (80) présente au moins un coulisseau de déclenchement (82), au moins un levier de déclenchement (16) s'appuyant sur le boîtier (10) et un élément de verrouillage (95) retenant la barre de traction (61) dans la position de verrouillage,
- le ou les leviers de déclenchement (16) sont déplaçables par un mouvement de coulissement longitudinal ou de pivotement du coulisseau de déclenchement (82) de telle sorte que l'élément de verrouillage (95) déverrouille la barre de traction (61) et déplace la portion d'appui (65) depuis la surface d'appui (37).

2. Injecteur à usage unique selon la revendication 1, **caractérisé en ce que** la barre de traction (61) forme conjointement avec la portion d'appui (65) un crochet de traction (62) qui vient en prise dans une position de verrouillage en s'appuyant par-dessus l'arête du boîtier (36).

3. Injecteur à usage unique selon la revendication 2, **caractérisé en ce qu'**au moins un groupe de deux ou de plus de deux crochets de traction (62) est disposé sur le poinçon d'actionnement de piston (60).

4. Injecteur à usage unique selon la revendication 2, **caractérisé en ce qu'**un élément de verrouillage (95) est disposé sur le boîtier (10) ou sur l'unité de déclenchement (80), lequel fixe le ou les crochets de traction (62) dans une position de verrouillage.

5. Injecteur à usage unique selon la revendication 1, **caractérisé en ce que** lors de l'utilisation de plusieurs leviers de déclenchement (16), ceux-ci sont disposés sur ou contre une bague (20).

6. Injecteur à usage unique selon au moins l'une quelconque des revendications précédentes, **caractérisé en ce que** le coulisseau de déclenchement (82) forme conjointement avec le ou les leviers de déclenchement (16) un mécanisme à cale coulissant, et lors d'un mouvement longitudinal du coulisseau de déclenchement (82), l'élément de verrouillage (95) subissant forcément un mouvement à l'encontre dudit mouvement longitudinal, tandis que les crochets de traction (62) sont déplacés transversalement à l'axe médian (5) de l'injecteur à usage unique.

7. Injecteur à usage unique selon la revendication 2, **caractérisé en ce que** dans le cas d'un poinçon d'actionnement de piston (60) ayant au moins deux crochets de traction (62), les centres de gravité des portions d'appui (65) des crochets de traction (62), du fait de leur fabrication, dans l'état non déformé se situent plus à l'écart les uns des autres que dans le cas d'un poinçon d'actionnement de piston (60) non déclenché, incorporé dans l'injecteur à usage unique.
